# EUROPEAN PATENT APPLICATION

(11) **EP 4 737 434 A2**
(43) Date of publication of application: **06.05.2026**
(21) Application number: 25208940.4
(22) Date of filing: 15.10.2025
(51) Int. Cl.: C07C 45/68, C07C 49/603, C07C 49/713, C07C 227/18, C07C 229/26, C07C 269/04, C07C 271/22, C07C 269/06

(54) **METHOD FOR PREPARING FMOC-LYS(IVDDE)-OH**

(30) Priority: 29.10.2024 CN 202411525001
(71) Applicant: Sichuan Shifang Sangao Biochemical Industrial Co., Ltd., Chengdu, Sichuan 618415 (CN)
(72) Inventor: LIANG, Hongguo, Chengdu, 618415 (CN); FANG, Xiaolong, Chengdu, 618415 (CN); LI, Qi, Chengdu, 618415 (CN); WAN, Xin, Chengdu, 618415 (CN); YANG, Jian, Chengdu, 618415 (CN); TIAN, Mingcheng, Chengdu, 618415 (CN); LI, Jiaxun, Chengdu, 618415 (CN)
(74) Representative: V.O.

(57) **Abstract**

The present disclosure provides a method for preparing Fmoc-Lys(Ivdde)-OH, comprising steps of: reacting dimedone with isovaleric acid in the presence of a condensing agent to obtain an intermediate compound; then reacting the intermediate compound with N-alpha-tert-butoxycarbonyl-L-Lysine in the presence of an organic base to obtain an intermediate; then reacting the intermediate with an acid to obtain another intermediate; and finally reacting the another intermediate with N-(9-fluorenylmethoxycarbonyloxy)succinimide in the presence of an inorganic base to obtain Fmoc-Lys(Ivdde)-OH. The method provided by the present disclosure uses dimedone as a starting material to synthesize Fmoc-Lys(Ivdde)-OH via a four-step reaction comprising two condensation reactions, a Boc deprotection reaction, and an Fmoc protection reaction. The method simplifies the process, features simple operations and mild reaction conditions, and reduces the difficulty of industrial production. Furthermore, the method does not involve expensive raw materials, which is beneficial for controlling production costs, and a product is obtained with a high purity and a high yield.

## Description

### FIELD

The present disclosure relates to the field of pharmaceutical synthesis technology, and more specifically relates to a method for preparing Fmoc-Lys(Ivdde)-OH.

### BACKGROUND

Fmoc-Lys(Ivdde)-OH is a common intermediate in the synthesis of peptides such as semaglutide, tirzepatide, and the like. The full chemical name of Fmoc-Lys(Ivdde)-OH is N-(9-fluorenylmethoxycarbonyl)-N'-[1-(4,4-dimethyl-2,6-dioxocyclohexylidene)-3-methylbutyl]-L-lysine, with a molecular formula of C₃₄H₄₂N₂O₆, a CAS number of 204777-78-6, and a structural formula of:

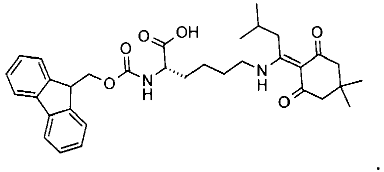

The prior art (By Chhabra, Siri Ram; et al. Tetrahedron Letters (1998), 39(12), 1603-1606) discloses a method for preparing Fmoc-Lys(Ivdde)-OH. This method involves reacting Fmoc-Lys, Ivdde-OH and trifluoroacetic acid in ethanol under reflux for 60 h, followed by a post-process to obtain Fmoc-Lys(Ivdde)-OH. The reaction process is as follows:

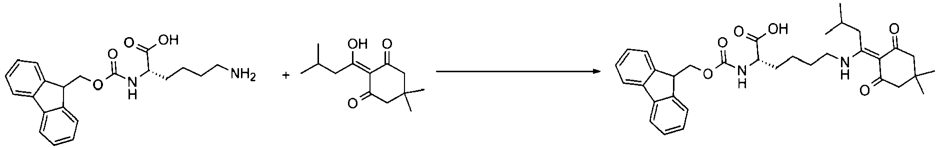

However, in the above reaction process, trifluoroacetic acid has strong corrosiveness, and the reaction is carried out at high temperature for a long time, resulting in high safety risks and poor suitability for large-scale production.

### SUMMARY

In view of the foregoing, the purpose of the present disclosure is to provide a method for preparing Fmoc-Lys(Ivdde)-OH. The method provided in the present disclosure features a simple process, a mild reaction condition, a low cost, and an implementability of industrial production.

The present disclosure provides a method for preparing Fmoc-Lys(Ivdde)-OH, comprising steps of:
a) reacting dimedone with isovaleric acid in the presence of a condensing agent to obtain an intermediate compound Ivdde-OH as represented by formula (3); 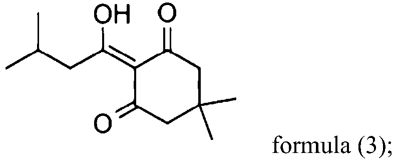
b) reacting the intermediate compound Ivdde-OH as represented by formula (3) with N-alpha-tert-butoxycarbonyl-L-Lysine in the presence of an organic base to obtain an intermediate Boc-Lys(Ivdde)-OH as represented by formula (5); 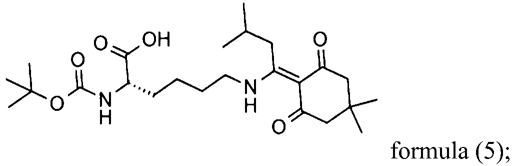
c) reacting the intermediate Boc-Lys(Ivdde)-OH as represented by formula (5) with an acid to obtain an intermediate Lys(Ivdde)-OH as represented by formula (6); 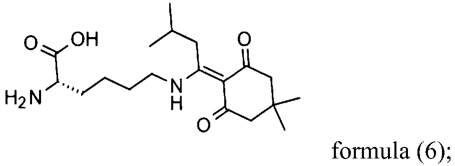
d) reacting the intermediate Lys(Ivdde)-OH as represented by formula (6) with N-(9-fluorenylmethoxycarbonyloxy)succinimide in the presence of an inorganic base to obtain Fmoc-Lys(Ivdde)-OH as represented by formula (8);

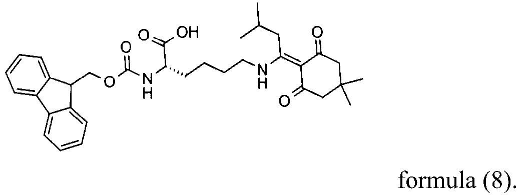

In some specific embodiments, in step a), the condensing agent is selected from the group consisting of 1-(3-dimethylaminopropyl)-3-ethylcarbodiimide hydrochloride, dicyclohexylcarbodiimide and a mixture thereof;
dimedone, isovaleric acid, and the condensing agent have a mole ratio of 1:(0.8 to 1.3):(1 to 1.5).

In some specific embodiments, in step a), the reaction is performed in a medium selected from the group consisting of dichloromethane, acetone, and tetrahydrofuran;
the reaction is performed at room temperature for 12 h to 16 h.

In some specific embodiments, in step b), the organic base is selected from the group consisting of triethylamine, N, N-diisopropylethylamine and a mixture thereof;
N-alpha-tert-butoxycarbonyl-L-Lysine, Ivdde-OH and the organic base have a mole ratio of 1:(1 to 1.5):(1.1 to 2.0).

In some specific embodiments, in step b), the reaction is performed in a medium selected from the group consisting of acetonitrile, methanol, and ethanol;
the reaction is performed at 65°C to 75°C for 12 h to 14 h.

In some specific embodiments, in step c), the acid is hydrochloric acid;
Boc-Lys(Ivdde)-OH and the acid have a mole ratio of 1:(5 to 8).

In some specific embodiments, in step c), the reaction is performed at room temperature for 6 h to 8 h.

In some specific embodiments, in step d), the inorganic base is selected from the group consisting of sodium carbonate, sodium bicarbonate, potassium carbonate and a mixture thereof.

In some specific embodiments, in step d), Lys(Ivdde)-OH, N-(9-fluorenylmethoxycarbonyloxy)succinimide, and the inorganic base have a mole ratio of 1:(0.7 to 1.0):(1.2 to 1.5).

In some specific embodiments, in step d), the reaction is performed in a medium selected from the group consisting of ethyl acetate, acetone, and tetrahydrofuran;
the reaction is performed at 25°C to 30°C for 3 h to 4 h.

The present disclosure provides a method for preparing Fmoc-Lys(Ivdde)-OH, comprising steps of: a) reacting dimedone with isovaleric acid in the presence of a condensing agent to obtain an intermediate compound Ivdde-OH as represented by formula (3); b) reacting the intermediate compound Ivdde-OH as represented by formula (3) with N-alpha-tert-butoxycarbonyl-L-Lysine in the presence of an organic base to obtain an intermediate Boc-Lys(Ivdde)-OH as represented by formula (5); c) reacting the intermediate Boc-Lys(Ivdde)-OH as represented by formula (5) with an acid to obtain an intermediate Lys(Ivdde)-OH as represented by formula (6); d) reacting the intermediate Lys(Ivdde)-OH as represented by formula (6) with N-(9-fluorenylmethoxycarbonyloxy)succinimide in the presence of an inorganic base to obtain Fmoc-Lys(Ivdde)-OH as represented by formula (8). The method provided by the present disclosure uses dimedone as a starting material to synthesize Fmoc-Lys(Ivdde)-OH via a four-step reaction comprising two condensation reactions, a Boc deprotection reaction, and an Fmoc protection reaction. The method simplifies the process, features simple operations and mild reaction conditions, and reduces the difficulty of industrial production. Furthermore, the method does not involve expensive raw materials, which is beneficial for controlling production costs, and a product is obtained with a high purity and a high yield. Experimental results show that the Fmoc-Lys(Ivdde)-OH prepared by the method provided in the present disclosure has a purity of over 99% and a yield of over 75% (calculated based on Boc-Lys).

### BRIEF DESCRIPTION OF DRAWINGS

FIG. 1 is an HPLC chromatogram of Fmoc-Lys(Ivdde)-OH prepared in example 2 provided in the present disclosure;
FIG. 2 is an infrared spectrum of Fmoc-Lys(Ivdde)-OH prepared in example 2 provided in the present disclosure;
FIG. 3 is a nuclear magnetic resonance spectrum of Fmoc-Lys(Ivdde)-OH prepared in example 2 provided in the present disclosure.

### DETAILED DESCRIPTION

The technical solutions of the present disclosure will be described clearly and completely with reference to some examples of the present disclosure as follows. It should be understood that the described examples are merely some, and not all, of the possible examples of the present disclosure. Based on the examples in the present disclosure, all other examples obtained by a person of ordinary skill in the art without applying inventive effort shall fall within the scope of protection of the present disclosure.

In the description of the present disclosure, it is to be understood that the orientational or positional relationships indicated by terms such as "center", "longitudinal", "transverse", "length", "width", "thickness", "upper", "lower", "front", "rear", "left", "right", "vertical", "horizontal", "top", "bottom", "inner", "outer", "clockwise", "counterclockwise", and the like, are based on the orientational or positional relationships shown in the accompanying drawings. These terms are used merely for convenience in describing the present disclosure and to simplify the description, and are not intended to indicate or imply that the referenced apparatus or element must have a particular orientation, or be constructed and operated in a particular orientation. Therefore, they should not be construed as limiting the present disclosure.

Furthermore, the terms "first" and "second" are used for descriptive purposes only and are not to be construed as indicating or implying any relative importance or implicitly indicating the number of technical features indicated. Thus, a feature defined by "first" or "second" may explicitly or implicitly include one or more of said features. In the description of the present disclosure, the meaning of "a plurality of" is two or more, unless otherwise explicitly and specifically defined. Moreover, the terms "mounted", "connected", and "coupled" should be understood in a broad sense. For example, a connection may be a fixed connection, a detachable connection, or an integral connection; it may be a mechanical connection or an electrical connection; it may be a direct connection or an indirect connection through an intermediary medium, or it may be the internal communication between two elements. For a person of ordinary skill in the art, the specific meanings of the aforementioned terms in the present disclosure can be understood according to the specific context.

The present disclosure provides a method for preparing Fmoc-Lys(Ivdde)-OH, comprising steps of:
a) reacting dimedone with isovaleric acid in the presence of a condensing agent to obtain an intermediate compound Ivdde-OH as represented by formula (3); 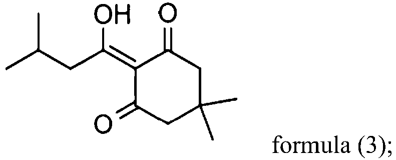
b) reacting the intermediate compound Ivdde-OH as represented by formula (3) with N-alpha-tert-butoxycarbonyl-L-Lysine in the presence of an organic base to obtain an intermediate Boc-Lys(Ivdde)-OH as represented by formula (5); 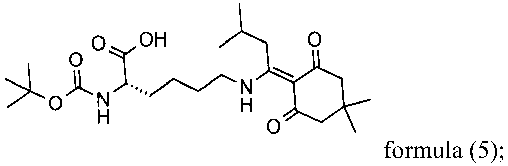
c) reacting the intermediate Boc-Lys(Ivdde)-OH as represented by formula (5) with an acid to obtain an intermediate Lys(Ivdde)-OH as represented by formula (6); 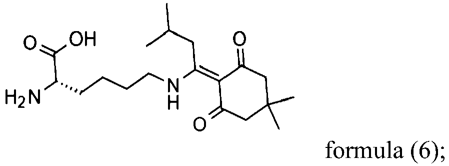
d) reacting the intermediate Lys(Ivdde)-OH as represented by formula (6) with N-(9-fluorenylmethoxycarbonyloxy)succinimide in the presence of an inorganic base to obtain Fmoc-Lys(Ivdde)-OH as represented by formula (8);

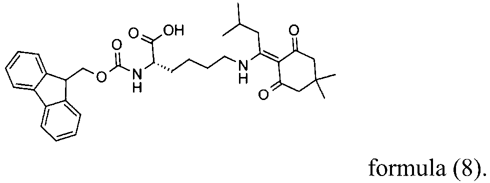

The present disclosure uses dimedone as a starting material to synthesize Fmoc-Lys(Ivdde)-OH via a four-step reaction comprising two condensation reactions, a Boc deprotection reaction, and an Fmoc protection reaction. The method simplifies the process, features simple operations and mild reaction conditions, and reduces the difficulty of industrial production. Furthermore, the method does not involve expensive raw materials, which is beneficial for controlling production costs, and yields Fmoc-Lys(Ivdde)-OH with a high purity and a high yield.

In the present disclosure, dimedone and isovaleric acid are used as starting materials, which are reacted in the presence of a condensing agent to produce the intermediate compound Ivdde-OH, as represented by formula (3). The reaction process is as follows:

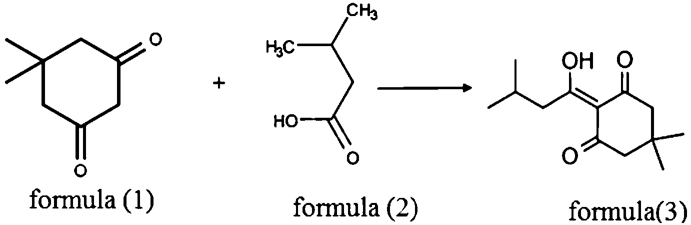

Dimedone has a structure as represented by formula (1), and there is no particular limitation on its source in the present disclosure. Isovaleric acid has a structure as represented by formula (2), and there is no particular limitation on its source in the present disclosure.

In some specific embodiments, in step a), the condensing agent includes but is not limited to 1-(3-dimethylaminopropyl)-3-ethylcarbodiimide hydrochloride (EDCI) or dicyclohexylcarbodiimide (DCC) or the like, and can be one or more of them. When the condensing agent comprises a combination of multiple substances, there is no particular limitation on their ratio in the present disclosure. In some specific embodiments, the condensing agent is selected from the group consisting of 1-(3-dimethylaminopropyl)-3-ethylcarbodiimide hydrochloride (EDCI), dicyclohexylcarbodiimide (DCC) and a mixture thereof.

In some specific embodiments, in step a), the reaction is performed in a medium, and the medium is an organic solvent. In some specific embodiments, the organic solvent is selected from the group consisting of ethyl acetate, acetone, and tetrahydrofuran.

In some specific embodiments, dimedone, isovaleric acid, and the condensing agent have a mole ratio of 1:(0.8 to 1.3):(1 to 1.5), preferably 1:1:1.1.

Specifically, in the present disclosure, dimedone, isovaleric acid and the organic solvent are first mixed thoroughly, then the condensing agent is added, and the reaction is performed, to obtain the intermediate compound Ivdde-OH as represented by formula (3). In some specific embodiments, the condensing agent is added at 15 to 20°C and the reaction is performed. In some specific embodiments, the reaction is performed at room temperature, for example, 25 to 35°C, preferably 28 to 32°C, for a duration of 12 h to 16 h, preferably 13 to 15 h.

After the reaction is completed, the obtained product is subjected to a post-process procedure, specifically comprising steps of:

adding water to the reaction system, allowing the mixture to stand for layer separation, and collecting the organic layer;

drying and concentrating the organic layer to obtain the intermediate compound Ivdde-OH as represented by formula (3).

There is no particular limitation on method of the drying step in the present disclosure; for example, drying can be conducted using sodium sulfate. After drying and concentrating, the intermediate compound Ivdde-OH as represented by formula (3) can be obtained, which can be used directly in the next step without further purification.

After the intermediate compound Ivdde-OH as represented by formula (3) is obtained, it is reacted with N-alpha-tert-butoxycarbonyl-L-Lysine in the presence of an organic base to obtain the intermediate Boc-Lys(Ivdde)-OH as represented by formula (5). The reaction process is as follows:

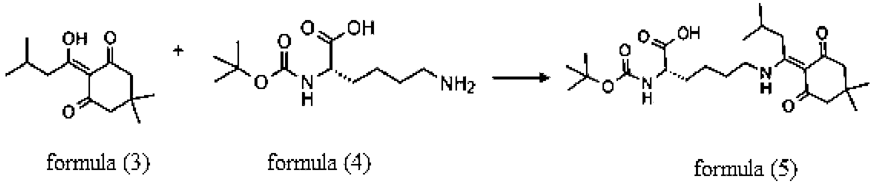

N-alpha-tert-butoxycarbonyl-L-Lysine (Boc-Lys) has a structure as represented by formula (4). There is no particular limitation on its source in the present disclosure, and it can be commercially available.

In some specific embodiments, the organic base includes but is not limited to triethylamine or N, N-diisopropylethylamine, and can be one or more of them. When the organic base is a combination of them, there is no particular limitation on their ratio in the present disclosure. In some specific embodiments, the organic base is selected from the group consisting of triethylamine, N, N-diisopropylethylamine and a mixture thereof.

In some specific embodiments, in step b), the reaction is performed in a medium, and the medium is an organic solvent. In some specific embodiments, the organic solvent is selected from the group consisting of acetonitrile, methanol, and ethanol.

In some specific embodiments, Boc-Lys, Ivdde-OH, and the organic base have a mole ratio of 1:(1 to 1.5):(1.1 to 2.0), preferably 1:1.1:1.5.

Specifically, in the present disclosure, Boc-Lys, Ivdde-OH, the organic base, and the organic solvent are mixed thoroughly, then heated and reacted under reflux to obtain the intermediate Boc-Lys(Ivdde)-OH as represented by formula (5). In some specific embodiments, the reaction is performed at a temperature of 65°C to 75°C for 12 h to 14 h. In some specific embodiments, the reaction is performed under stirring.

After the reaction is completed, the obtained product is subjected to a post-process procedure, specifically comprising steps of:
concentrating the reaction system to dryness, cooling, and adding water, an organic solvent and an acid, allowing the mixture to stand for layer separation, and collecting the organic layer;
washing, drying, concentrating, crystallizing, filtering, and drying again the organic layer to obtain the intermediate Boc-Lys(Ivdde)-OH as represented by formula (5).

In the present disclosure, the reaction system is first concentrated to dryness, and preferably cooled to room temperature before adding water, an organic solvent and an acid; wherein, the organic solvent can be ethyl acetate and the like; the acid is added to adjust the pH of the system to 4 to 5; there is no particular limitation on type of the acid in the present disclosure, for example, the acid can be citric acid. After standing for layer separation, there is no particular limitation on the method of washing the collected organic layer in the present disclosure; for example, the organic layer can be washed with saturated sodium chloride solution three times. There is no particular limitation on method of the drying step in the present disclosure; for example, sodium sulfate can be used for drying. After drying, the organic layer is filtered, and the obtained filtrate is concentrated until a large amount of solid precipitates, and cooled again and crystallized. Preferably, in the present disclosure, a solvent such as petroleum ether is added for crystallization for a duration of 1 h to 3 h. The obtained product is filtered and dried again to obtain the intermediate Boc-Lys(Ivdde)-OH as represented by formula (5). There is no particular limitation on the re-drying step in the present disclosure; for example, it can be performed at a temperature of 50°C to 55°C.

After the intermediate Boc-Lys(Ivdde)-OH as represented by formula (5) is obtained, it is reacted with an acid to obtain the intermediate Lys(Ivdde)-OH as represented by formula (6). The reaction process is as follows:

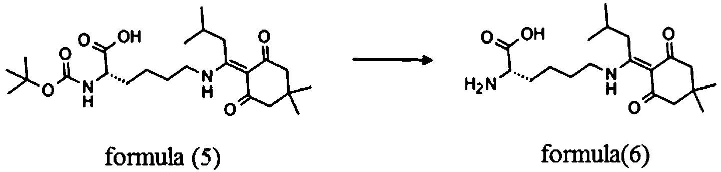

In some specific embodiments, the acid is hydrochloric acid, which is used to remove the Boc protecting group.

In some specific embodiments, Boc-Lys(Ivdde)-OH and the acid have a mole ratio of 1:(5 to 8), preferably 1:7.

In some specific embodiments, the reaction is performed in a medium, and the medium is an organic solvent, including but not limited to tetrahydrofuran and the like.

Specifically, in the present disclosure, the organic solvent and the acid are mixed and are then mixed and reacted with the intermediate Boc-Lys(Ivdde)-OH as represented by formula (5), to obtain the intermediate Lys(Ivdde)-OH as represented by formula (6). In some specific embodiments, the reaction is performed at room temperature, for example 20°C to 25°C, for 6 h to 8 h.

After the reaction is completed, a base is added to the obtained reaction system to adjust the pH to 6 to 7. After standing for layer separation, the aqueous layer is collected to obtain the intermediate Lys(Ivdde)-OH as represented by formula (6), which can be used directly in the next step without further purification.

After the intermediate Lys(Ivdde)-OH as represented by formula (6) is obtained, it is reacted with N-(9-fluorenylmethoxycarbonyloxy)succinimide in the presence of an inorganic base, to obtain the Fmoc-Lys(Ivdde)-OH as represented by formula (8). The reaction process is as follows:

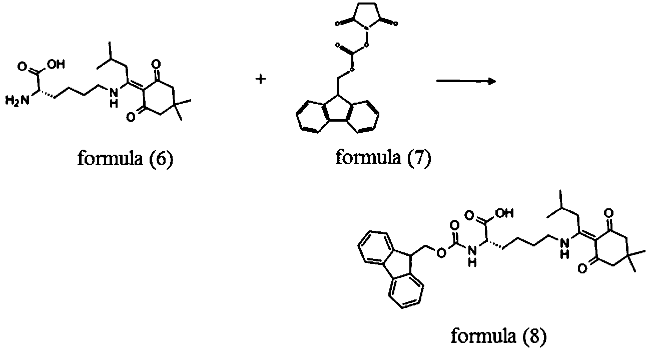

N-(9-fluorenylmethoxycarbonyloxy)succinimide (Fmoc-Osu) has a structure as represented by formula (7), and there is no particular limitation on its source in the present disclosure.

In some specific embodiments, the inorganic base includes but is not limited to sodium carbonate, sodium bicarbonate, potassium carbonate or the like, and can be one or more of them. When the inorganic base is a combination of them, there is no particular limitation on their ratio in the present disclosure.

In some specific embodiments, the reaction is performed in an organic solvent, which includes but is not limited to ethyl acetate, acetone or tetrahydrofuran.

In some specific embodiments, Lys(Ivdde)-OH, Fmoc-Osu, the inorganic base have a mole ratio of 1:(0.7 to 1.0):(1.2 to 1.5), preferably 1:0.8:1.4.

Specifically, in the present disclosure, the aqueous solution of Lys(Ivdde)-OH, the organic solvent, and the inorganic base are mixed thoroughly, then Fmoc-Osu is added and the reaction is performed to obtain Fmoc-Lys(Ivdde)-OH as represented by formula (8). In some specific embodiments, Fmoc-Osu is preferably added at a temperature of 25°C to 30°C, and the reaction is preferably performed at a temperature of 25°C to 30°C. A too low reaction temperature can result in a prolonged reaction duration; while a too high reaction temperature can result in increased impurities which are hard to remove. In some specific embodiments, the reaction is performed for a duration of 3 h to 4 h. In some specific embodiments, the reaction is performed under stirring.

After the reaction is completed, it is preferred to perform a post-process procedure, specifically comprising steps of:
adding an acid to the reaction system to adjust the pH to 2 to 3, performing layer separation and collecting the organic layer;
washing, drying, concentrating, crystallizing, centrifuging, and drying again the organic layer to obtain the intermediate Fmoc-Lys(Ivdde)-OH as represented by formula (8).

There is no particular limitation on the acid in the present disclosure; for example, it can be concentrated hydrochloric acid. There is no particular limitation on method of the washing step in the present disclosure; for example, saturated sodium chloride solution can be used for washing three times. There is no particular limitation on the method of drying step in the present disclosure; for example, sodium sulfate can be used for drying. There is no particular limitation on method of the crystallizing step in the present disclosure; for example, the temperature can be cooled to 20°C to 25°C and then an organic solvent is added for crystallizing; wherein the organic solvent includes but is not limited to petroleum ether, and the crystallizing duration is preferred to be 1 h to 3 h. There is no particular limitation on the drying step after centrifuging in the present disclosure; for example, drying can be performed at a temperature of 50°C to 55°C.

After the reaction is completed, the obtained solid is subjected to HPLC analysis, infrared analysis, and nuclear magnetic resonance analysis. The results indicate that the product is Fmoc-Lys(Ivdde)-OH, with a purity over 99% and a yield over 75% (calculated based on Boc-Lys).

The following examples are provided to further illustrate the method for preparing Fmoc-Lys(Ivdde)-OH disclosed in the present disclosure.

### Example 1

### (1) Preparation of Ivdde-OH:

To a 1 L three-necked flask, 650 g of dichloromethane was added, followed by 100 g of dimedone as represented by formula (1) and 73 g of isovaleric acid as represented by formula (2) under stirring. The mixture was stirred until completely dissolved. Subsequently, 150 g of 1-(3-dimethylaminopropyl)-3-ethylcarbodiimide hydrochloride (EDCI) was added while the temperature was controlled at 15°C to 20°C. After the addition was completed, the temperature was maintained at 25°C to 30°C and the mixture was reacted for 13 h until the raw material dimedone had completely reacted, as indicated by thin-layer chromatography (TLC). To the system, 200 g of water were added, and the mixture was stirred until clear, and then allowed to stand for layer separation. The dichloromethane layer was collected, dried over sodium sulfate, and filtered. The filtrate was concentrated to dryness to obtain 121 g of an oily substance, namely Ivdde-OH as represented by formula (3), with a yield of 81.2%. The product was used directly in the next step without analysis or purification. The reaction step was as follows:

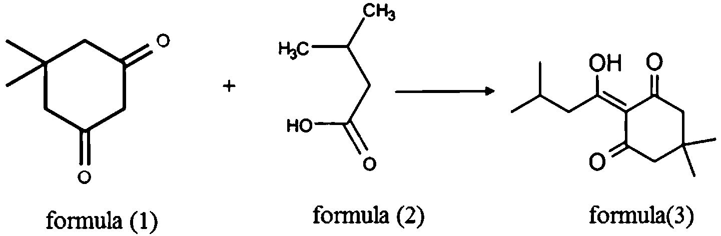

### (2) Preparation of Boc-Lys(Ivdde)-OH

To a 2 L three-necked flask, 726 g of methanol were added, followed by 121 g of N-alpha-tert-butoxycarbonyl-L-Lysine (Boc-Lys) as represented by formula (4), 121 g of Ivdde-OH prepared in step (1) and 95 g of N, N-diisopropylethylamine under stirring. The mixture was heated to an internal temperature of 70°C to 75°C and reacted for 13 h until the raw material Boc-Lys had completely reacted, as indicated by TLC. The methanol in the reaction system was concentrated to dryness. The system was cooled to 20°C to 30°C, and 200 g of ethyl acetate and 200 g of water were added to the reaction mixture. The system was acidified with citric acid to a pH of 4 to 5, and then allowed to stand for layer separation. The ethyl acetate layer was collected, washed three times with saturated sodium chloride solution, dried over sodium sulfate, filtered, and concentrated until a large amount of solid precipitated. The mixture was cooled to 20°C to 25°C, 200 g of petroleum ether was added, and the mixture was crystallized for 2 h. The product was filtered and dried at 50°C to 55°C to obtain 198 g of the solid intermediate Boc-Lys(Ivdde)-OH, with a yield of 89% and an HPLC purity of 99.18%. The reaction process was as follows:

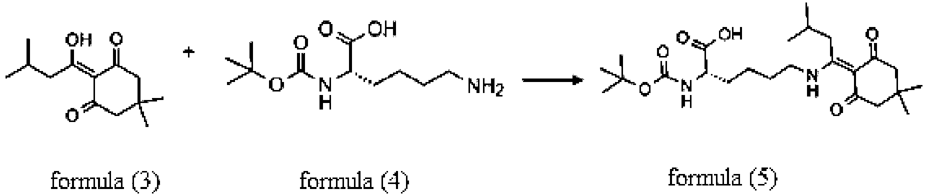

### (3) Preparation of Lys(Ivdde)-OH

To a 2 L three-necked flask, 560 g of tetrahydrofuran and 612 g of 6 N hydrochloric acid were added, followed by 198 g of Boc-Lys(Ivdde)-OH prepared in step (2) under stirring. The temperature was maintained at 20°C to 25°C and the mixture was reacted for 6 h until the raw material Boc-Lys(Ivdde)-OH had completely reacted, as indicated by TLC. Sodium carbonate was slowly added to the system until the pH was adjusted to 6 to 7, and then the mixture was allowed to stand for layer separation. The aqueous layer was collected to obtain the aqueous solution of Lys(Ivdde)-OH as represented by formula (6), which was used directly in the next step without purification. The reaction process was as follows:

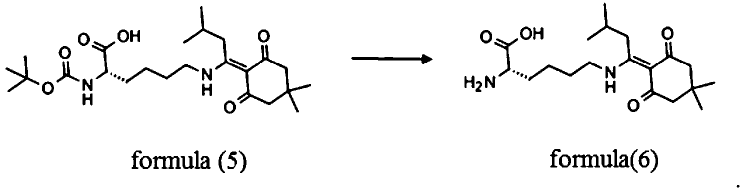

### (4) Preparation of Fmoc-Lys(Ivdde)-OH

To a 2 L three-necked flask, the aqueous solution of Lys(Ivdde)-OH prepared in step (3) was added, followed by 750 g of ethyl acetate and 118 g of sodium carbonate, and the mixture was then stirred thoroughly. The temperature was maintained at 25°C to 30°C, and 130 g of N-(9-fluorenylmethoxycarbonyloxy)succinimide (Fmoc-Osu) was added to the mixture, which was reacted for 3 h until Fmoc-Osu had completely reacted, as indicated by TLC. The pH of the system was adjusted to 2 to 3 with concentrated hydrochloric acid. The mixture was allowed to stand for layer separation. The ethyl acetate layer was collected, washed three times with saturated sodium chloride solution, dried over sodium sulfate, filtered and concentrated until a large amount of solid precipitated. The mixture was cooled to 20°C to 25°C, 350 g of petroleum ether was added, and the mixture was crystallized for 2 h. The product was filtered and dried at a temperature of 50°C to 55°C to obtain 182 g of the solid intermediate Fmoc-Lys(Ivdde)-OH, with a yield of 82.18% and an HPLC purity of 99.69%. The reaction process was as follows:

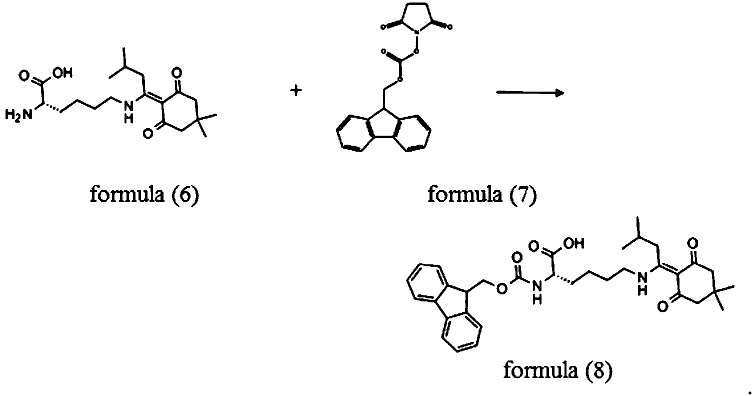

### Example 2

### (1) Preparation of Ivdde-OH:

To a 500 L reaction kettle, 150 kg of dichloromethane was added, followed by 23 kg of dimedone and 16.8 kg of isovaleric acid under stirring. The mixture was stirred until completely dissolved. Subsequently, 37.1 kg of dicyclohexylcarbodiimide (DCC) was added while the temperature was maintained at 15°C to 20°C. After the addition was completed, the temperature was maintained at 25°C to 30°C and the mixture was reacted for 14 h until the raw material dimedone had completely reacted, as indicated by TLC. The insoluble substance in the system (DCU) was filtered out. The filtrate was collected and concentrated to dryness to obtain 31 kg of an oily substance with a yield of 84.23%. The product was used directly in the next step without analysis or purification.

### (2) Preparation of Boc-Lys(Ivdde)-OH

To a clean and anhydrous 500 L reaction kettle, 186 kg of ethanol was added, followed by 31 kg of Boc-Lys, 31 kg of Ivdde-OH prepared in step (1) and 19 kg of triethylamine under stirring. The mixture was heated to an internal temperature of 70°C to 75°C and reacted for 13 h until the raw material Boc-Lys had completely reacted, as indicated by TLC. The ethanol in the reaction system was concentrated to dryness. The system was cooled to 20°C to 30°C, and 100 kg of ethyl acetate and 100 kg of water were added to the reaction mixture. The system was acidified with citric acid to a pH of 4 to 5, and then allowed to stand for layer separation. The ethyl acetate layer was collected, washed three times with saturated sodium chloride solution, dried over sodium sulfate, filtered, and concentrated until a large amount of solid precipitated. The mixture was cooled to 20°C to 25°C, 100 kg of petroleum ether was added, and the mixture was crystallized for 2 h. The product was centrifuged and dried at 50°C to 55°C to obtain 53 kg of the solid intermediate Boc-Lys(Ivdde)-OH, with a yield of 93% and an HPLC purity of 99.28%. The HPLC data are shown in the following table:

| Peak No. | Peak Type | Retention Time (min) | Area (mAu·s) | Area (%) | Height (mAu) | Concentration (-) |
|---|---|---|---|---|---|---|
| 1 | single peak | 3.347 | 5.208 | 0.031 | 1.009 | 5.208 |
| 2 | single peak | 3.723 | 1.613 | 0.010 | 0.334 | 1.613 |
| 3 | single peak | 4.078 | 19.051 | 0.114 | 2.523 | 19.051 |
| 4 | single peak | 4.668 | 5.067 | 0.030 | 0.624 | 5.067 |
| 5 | single peak | 4.937 | 23.112 | 0.138 | 3.685 | 23.112 |
| 6 | single peak | 5.484 | 3.473 | 0.021 | 0.463 | 3.473 |
| 7 | single peak | 5.983 | 1.862 | 0.011 | 0.208 | 1.862 |
| 8 | overlapping peak | 7.037 | 16612.320 | 99.283 | 1491.489 | 16612.320 |
| 9 | overlapping peak | 7.722 | 11.500 | 0.069 | 0.910 | 11.500 |
| 10 | single peak | 8.662 | 5.333 | 0.032 | 0.623 | 5.333 |
| 11 | overlapping peak | 9.711 | 17.738 | 0.106 | 1.353 | 17.738 |
| 12 | overlapping peak | 10.080 | 3.929 | 0.023 | 0.283 | 3.929 |
| 13 | single peak | 12.369 | 8.786 | 0.053 | 0.551 | 8.786 |
| 14 | single peak | 15.678 | 13.226 | 0.079 | 0.810 | 13.226 |
| Total | | | 16732.230 | 100.000 | 1504.864 | 16732.230 |

### (3) Preparation of Lys(Ivdde)-OH

To a clean 500 L reaction kettle, 150 kg of tetrahydrofuran and 164 kg of 6 N hydrochloric acid were added, followed by 53 kg of Boc-Lys(Ivdde)-OH prepared in step (2) under stirring. The temperature was maintained at 20°C to 25°C and the mixture was reacted for 7 h until the raw material Boc-Lys(Ivdde)-OH had completely reacted, as indicated by TLC. Sodium carbonate was slowly added to the system until the pH was adjusted to 6 to 7, and then the mixture was allowed to stand for layer separation. The aqueous layer was collected to obtain the aqueous solution of Lys(Ivdde)-OH, which was used directly in the next step without purification.

### (4) Preparation of Fmoc-Lys(Ivdde)-OH

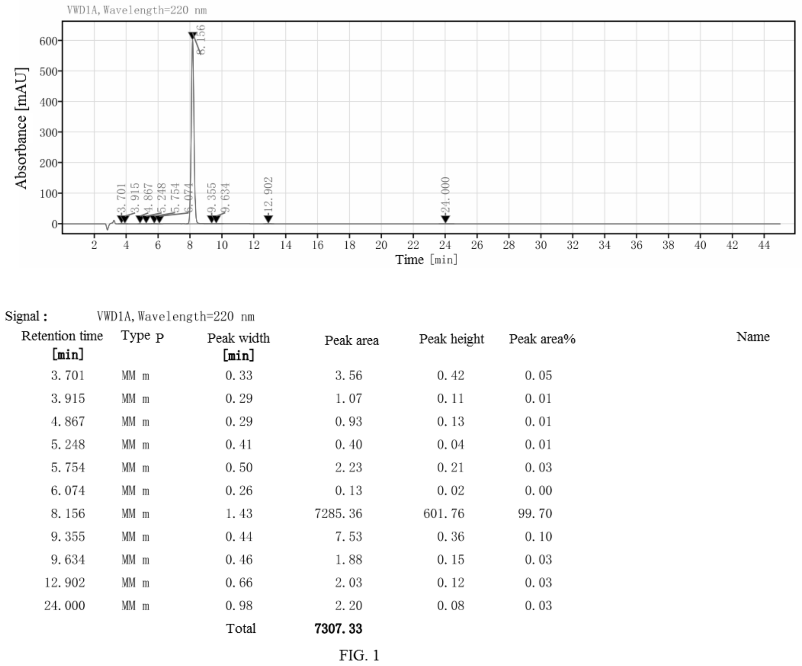

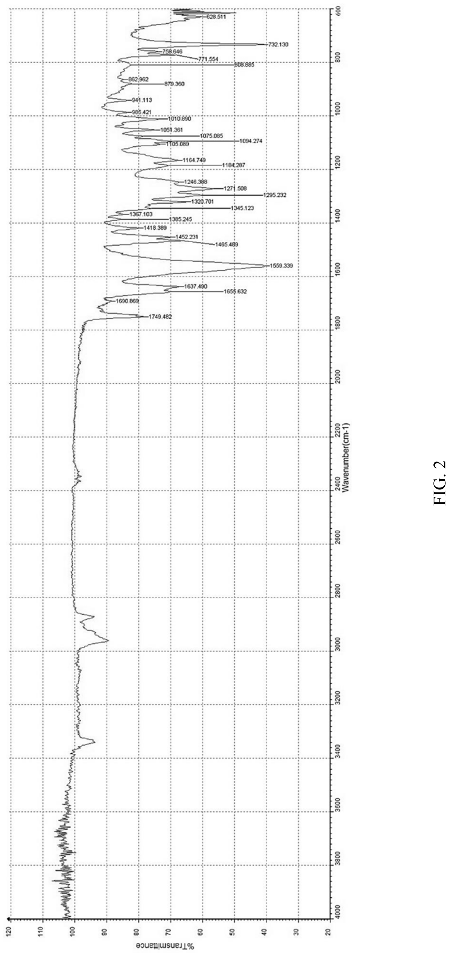

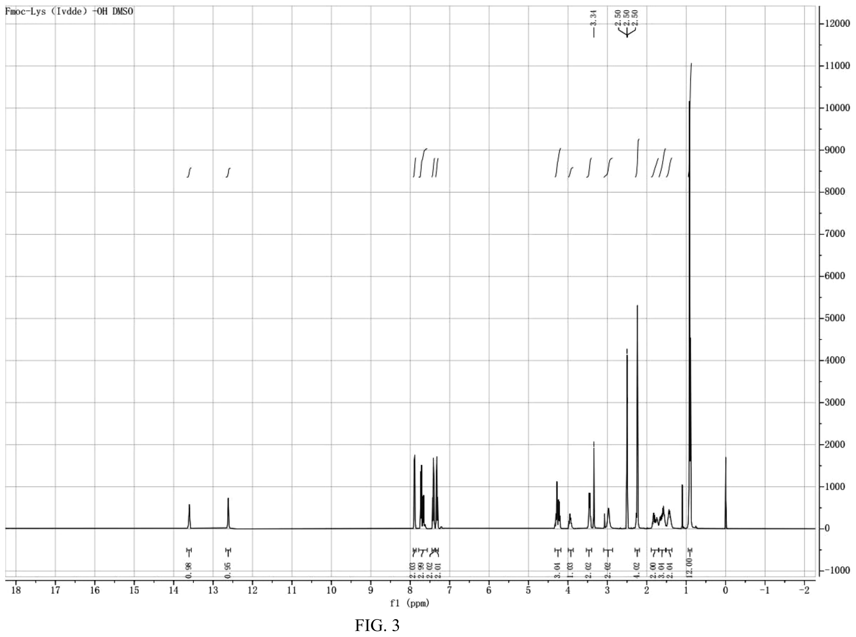

To a 1000 L reaction kettle, the aqueous solution of Lys(Ivdde)-OH prepared in step (3) was added, followed by 200 kg of ethyl acetate and 31.5 kg of sodium carbonate, and the mixture was then stirred thoroughly. The temperature was maintained at 25°C to 30°C, and 35 kg of Fmoc-Osu was added to the mixture, which was reacted for 3 h until Fmoc-Osu had completely reacted, as indicated by TLC. The pH of the system was adjusted to 2 to 3 with concentrated hydrochloric acid. Then the mixture was allowed to stand for layer separation. The ethyl acetate layer was collected, washed three times with saturated sodium chloride solution, dried over sodium sulfate, filtered and concentrated until a large amount of solid precipitated. The mixture was cooled to 20°C to 25°C, 50 kg of petroleum ether was added, and the mixture was crystallized for 2 h. The product was centrifuged and dried at a temperature of 50°C to 55°C to obtain 50 kg of the solid intermediate Fmoc-Lys(Ivdde)-OH, with a yield of 83.9% and an HPLC purity of 99.70%, as shown by Fig. 1, 2, and 3. FIG. 1 is an HPLC chromatogram of Fmoc-Lys(Ivdde)-OH prepared in example 2 provided in the present disclosure. FIG. 2 is an infrared spectrum of Fmoc-Lys(Ivdde)-OH prepared in example 2 provided in the present disclosure. FIG. 3 is a nuclear magnetic resonance spectrum of Fmoc-Lys(Ivdde)-OH prepared in example 2 provided in the present disclosure.

### Example 3

The preparation steps and technical parameters in the preparation method of Example 3 were the same as those in step (1) of Example 1, except that the type of the condensing agent was changed. The yields and purities of step (1) were shown in Table 1.

**Table 1**

| | Condensing Agent | Yield |
|---|---|---|
| Example 1 | EDCI | 81.2% |
| Example 3 | DCC | 84.23% |

### Example 4

The preparation steps and technical parameters in the preparation method of Example 4 were the same as those in step (2) of Example 1, except that the type of the organic solvent was changed. The yields and purities of step (2) were shown in Table 2.

**Table 2**

| | Organic Solvent | Reaction Time | Yield | Purity |
|---|---|---|---|---|
| Example 1 | methanol | 13 h | 89% | 99.18% |
| Example 2 | ethanol | 13 h | 93% | 99.28% |
| Example 4 | acetonitrile | 16 h | 85% | 98.56% |

### Examples 5 to 6 and Comparative Examples 1 to 2

The preparation steps and technical parameters in the preparation method of Examples 5 to 6 and Comparative Examples 1 to 2 were the same as those in step (2) of Example 1, except that the reaction temperature was changed. The yields and purities of step (2) were shown in Table 3.

**Table 3**

| | Reaction Temperature | Reaction Time | Yield | Purity |
|---|---|---|---|---|
| Example 1 | 70°C to 75°C | 13 h | 89% | 99.18% |
| Example 5 | 76°C to 80°C | 12 h | 85% | 99.06% |
| Example 6 | 65°C to 69°C | 16 h | 85% | 98.98% |
| Comparative example 1 | 50°C to 55°C | 34 h | 63% | 97.46% |
| Comparative example 2 | 30°C to 35°C | failed to completely react after 48 h | 36% | 68.53% |

### Examples 7 to 8

The preparation steps and technical parameters in the preparation method of Examples 7-8 were the same as those in step (4) of Example 1, except that the type of the inorganic base was changed. The yields and purities of step (4) were shown in Table 4.

**Table 4**

| | Inorganic Base | Reaction Time | Yield | Purity |
|---|---|---|---|---|
| Example 1 | sodium carbonate | 3 h | 82.18% | 99.69% |
| Example 7 | potassium carbonate | 2 h | 80.12% | 99.38% |
| Example 8 | sodium bicarbonate | 18 h | 78.3% | 99.45% |

### Examples 9 to 10

The preparation steps and technical parameters in the preparation method of Examples 9-10 were the same as those in step (4) of Example 1, except that the organic solvent was changed. The yields and purities of step (4) were shown in Table 5.

**Table 5**

| | Organic Solvent | Yield | Purity |
|---|---|---|---|
| Example 1 | ethyl acetate | 82.18% | 99.69% |
| Example 9 | acetone | 77.23% | 99.58% |
| Example 10 | tetrahydrofuran | 78.68% | 99.63% |

In summary, the present disclosure provides a method for preparing Fmoc-Lys(Ivdde)-OH, which is suitable for scaling up. This synthesis method requires simple equipment, demonstrates good reaction efficiency, has a relatively low production cost, and yields a final product with a purity of >99.0% and a yield of over 75% (calculated based on Boc-Lys).

The foregoing descriptions are merely preferred embodiments of the present disclosure. It should be noted that for those of ordinary skill in the art, several improvements and embellishments can be made without departing from the principles of the present disclosure. These improvements and embellishments should also be considered within the scope of protection of the present disclosure.

## Claims

1. A method for preparing Fmoc-Lys(Ivdde)-OH, comprising steps of:
a) reacting dimedone with isovaleric acid in the presence of a condensing agent to obtain an intermediate compound Ivdde-OH as represented by formula (3); 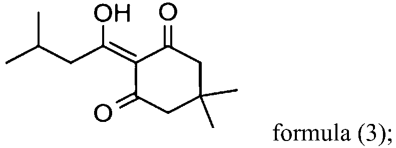
b) reacting the intermediate compound Ivdde-OH as represented by formula (3) with N-alpha-tert-butoxycarbonyl-L-Lysine in the presence of an organic base to obtain an intermediate Boc-Lys(Ivdde)-OH as represented by formula (5); 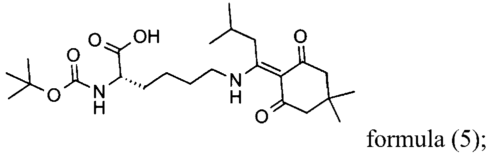
c) reacting the intermediate Boc-Lys(Ivdde)-OH as represented by formula (5) with an acid to obtain an intermediate Lys(Ivdde)-OH as represented by formula (6); 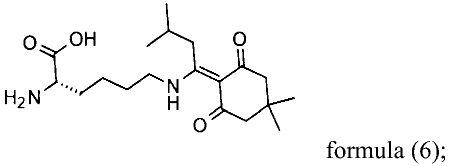
d) reacting the intermediate Lys(Ivdde)-OH as represented by formula (6) with N-(9-fluorenylmethoxycarbonyloxy)succinimide in the presence of an inorganic base to obtain Fmoc-Lys(Ivdde)-OH as represented by formula (8);

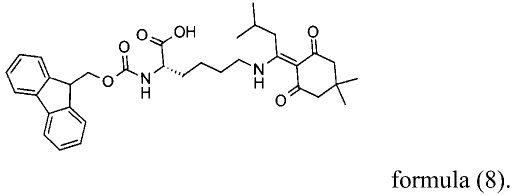

2. The method according to claim 1, wherein in step a), the condensing agent is selected from the group consisting of 1-(3-dimethylaminopropyl)-3-ethylcarbodiimide hydrochloride, dicyclohexylcarbodiimide and a mixture thereof;
the dimedone, the isovaleric acid, and the condensing agent have a mole ratio of 1:(0.8 to 1.3):(1 to 1.5).

3. The method according to claim 2, wherein in step a), the reaction is performed in a medium selected from the group consisting of dichloromethane, acetone, and tetrahydrofuran;
the reaction is performed at room temperature for 12 h to 16 h.

4. The method according to claim 1, wherein in step b), the organic base is selected from the group consisting of triethylamine, N, N-diisopropylethylamine and a mixture thereof;
N-alpha-tert-butoxycarbonyl-L-Lysine, Ivdde-OH and the organic base have a mole ratio of 1:(1 to 1.5):(1.1 to 2.0).

5. The method according to claim 4, wherein in step b), the reaction is performed in a medium selected from the group consisting of acetonitrile, methanol, and ethanol;
the reaction is performed at 65°C to 75°C for 12 h to 14 h.

6. The method according to claim 1, wherein in step c), the acid is hydrochloric acid; Boc-Lys(Ivdde)-OH and the acid have a mole ratio of 1:(5 to 8).

7. The method according to claim 6, wherein in step c), the reaction is performed at room temperature for 6 h to 8 h.

8. The method according to claim 1, wherein in step d), the inorganic base is selected from the group consisting of sodium carbonate, sodium bicarbonate, potassium carbonate and a mixture thereof.

9. The method according to claim 8, wherein in step d), Lys(lvdde)-OH, N-(9-fluorenylmethoxycarbonyloxy)succinimide, and the inorganic base have a mole ratio of 1:(0.7 to 1.0):(1.2 to 1.5).

10. The method according to claim 8, wherein in step d), the reaction is performed in a medium selected from the group consisting of ethyl acetate, acetone, and tetrahydrofuran;
the reaction is performed at 25°C to 30°C for 3 h to 4 h.
